# EUROPEAN PATENT APPLICATION

(11) **EP 0 539 625 A1**
(43) Date of publication of application: **05.05.1993**
(21) Application number: 91202787.7
(22) Date of filing: 28.10.1991
(51) Int. Cl.: C12Q 1/00, G01N 27/404

(54) **Electrochemical sensor for measuring the glucose content of glucose containing fluids**

(71) Applicant: Dräger Medical Electronics B.V., NL-5683 PK Best (NL)
(72) Inventor: Janssen, Leonard Johannes Joseph, NL-5671 ED Nuenen (NL); van Stroe, Saskia Anna Maria, NL-5632 DX Eindhoven (NL)
(74) Representative: de Bruijn, Leendert C.

(57) **Abstract**

Electrochemical sensor for measuring the glucose content fluids, containing very low oxygen content or no oxygen at all, comprising: a substrate (1) carrying on its passivated surface, a detection electrode (2), a counter electrode (3) cooperating with said detection electrode, a reference electrode (4), an oxygen producing electrode (5), and a second counter electrode (6) cooperating with said oxygen producing electrode. Furthermore the sensor comprises a glucose oxidase (G.O.D) containing hydrogel layer (7) covering said surface of the substrate respectively the electrodes arranged thereon, a membrane impermeable to glucose (8), having an opening or hole (10) rendering said membrane locally permeable for glucose, and a further membrane (9) permeable to oxygen, glucose and water but impermeable for components with high molecular weight in the glucose containing fluid. The arrangement of the various electrodes on the substrate surface and the location of the hole in said membrane is such that the direction of the glucose diffusion path (12) between said hole and the detection electrode opposite to the direction of the oxygen diffusion path (13) between the oxygen producing electrode and the detection electrode.

## Description

The invention relates to an electrochemical sensor for measuring the glucose content of fluids containing very low oxygen content or no oxygen at all, comprising:
- a substrate carrying on its passivated surface
   - a detection electrode
   - a counter electrode cooperating with said detection electrode
   - a reference electrode
- a glucose oxidase (G.O.D) containing hydrogel layer covering said surface of the substrate respectively the electrodes arranged thereon
- a membrane impermeable to glucose, having an opening or hole rendering said membrane locally permeable for glucose
- a further membrane permeable to oxygen, glucose and water but impermeable for components with high molecular weight in the glucose containing fluid.

An electrochemical sensor of the above described type is known from the European patent applications EP 0 363 504 and EP 0 396 788. The functioning of these prior art glucose sensors is based on the reaction of glucose with oxygen, whereby the glucose-oxidase enzyme (G.O.D.) is used as catalyzer. The reaction produces gluconolactone and hydrogen peroxide. At the detection electrode the hydrogen peroxide is oxidized or reduced resulting into an electrical current through the detection electrode which forms a measure for the glucose content in the measured fluid. More details about the reactions in the glucose sensor can be retrieved from the above-mentioned European patent applications as well as from the US patent US 4,492,622 and the article "......" by S. Gernet and others in Sensors and Actuators 18 (1989), pages 59-70.

If the sensor is to be used in an anaerobe environment or in an environment with very low oxygen content, then oxygen has to be produced in the sensor itself to obtain a reaction which produces a detectable electrical current. In the method, described in EP 0 396 788 use is made of a variable potential on the detection electrode to maintain a sufficient oxygen partial pressure. During a first period with a relatively low potential on the detection electrode the hydrogen peroxide is oxidized whereas during a second time period a relatively high potential is applied to the detection electrode whereby simultaneously the hydrogen peroxide is oxidized and the glucose reacts with the formed oxygen.

Because the diffusion coefficient of oxygen is significantly larger than the diffusion coefficient of glucose it is expected that the reaction of glucose with oxygen takes place in that part of the glucose-oxidase containing layer near to the hole through which the glucose enters the sensor. That implies that the major part of the formed hydrogen peroxide will leave the sensor through the same hole through which the glucose entered the sensor. Furthermore the distance between the actual reaction zone where the hydrogen peroxide is formed and the detection electrode is relatively large and as a consequence the electrical current developed in the detection electrode will be relatively small.

The diffusion mechanism of oxygen, glucose and hydrogen peroxide in sensors of the above-mentioned type, in which oxygen has to be formed within the sensor itself, is not well-defined and therefore it is hardly possible to provide a reasonable mathematical description. As a consequence the relation between the glucose content in the fluid to be measured and the detection current obtained in the detection electrode is very sensitive to the enzyme activity, the properties of the various membrane layers in the sensor and the concentration or concentration gradient of the oxygen in the sensor itself. Therefore none of the prior art sensors is well-suited for use in anaerobe fluids or fluids with a very low oxygen content.

An object of the invention is now to provide an electrochemical sensor for measuring the glucose content of glucose containing fluids in which means are present to produce oxygen within the sensor itself and whereby the configuration of the sensor as a whole is such that the reaction between glucose and oxygen takes place in a defined area adjacent to the detection electrode, so that a readily detectable current in the detection electrode can be obtained.

In agreement with this object the invention now provides an electrochemical sensor of the type identified in the first paragraph of this description, which is according to the invention characterized in that the substrate carries furthermore
- an oxygen producing electrode
- a second counter electrode cooperating with said oxygen producing electrode,
whereby the arrangement of the various electrodes on the substrate surface and the location of the hole in said membrane is such that the direction of the glucose diffusion path between said hole and the detection electrode is opposite to the direction of the oxygen diffusion path between the oxygen producing electrode and the detection electrode.

By selecting opposite directions for the oxygen diffusion flow on the one hand and the glucose diffusion flow on the other hand the actual reaction zone where the glucose reacts with the oxygen will be confined to a rather limited area just above the detection electrode.

If in a specific configuration of the electrochemical sensor the diffusion rate of the glucose and of the oxygen in the hydrogel layer are known, then it is preferred that the ratio of the distance between the hole and the detection electrode and the distance between the oxygen producing electrode and the detection electrode is at least substantially proportional to the ratio of the glucose diffusion rate and the oxygen diffusion rate in the hydrogel layer.

In a furthermore preferred embodiment the detection electrode is embodied as an array of subelectrodes extending with predetermined mutual distance perpendicular to the glucose and oxygen diffusion directions. With this embodiment it is not only possible to select the subelectrode from the array which provides the maximum detection current, but it is also possible to use more than one subelectrode and to measure various currents to obtain a profile of the amount of hydrogen peroxide formed in various parts of the reaction zone. In other words it is possible to obtain information about the reaction intensity in the reaction zone.

The invention will now be explained in more detail with reference to the attached drawings.

Figure 1 illustrates schematically a cross-section through an embodiment of the sensor according to the invention.

Figure 2 illustrates the relevant diffusion directions for glucose, oxygen and hydrogen peroxide.

Figure 3 illustrates a top view on the sensor of figure 1 whereby the two membrane layers and the hydrogel layer are removed to show the configuration of electrodes.

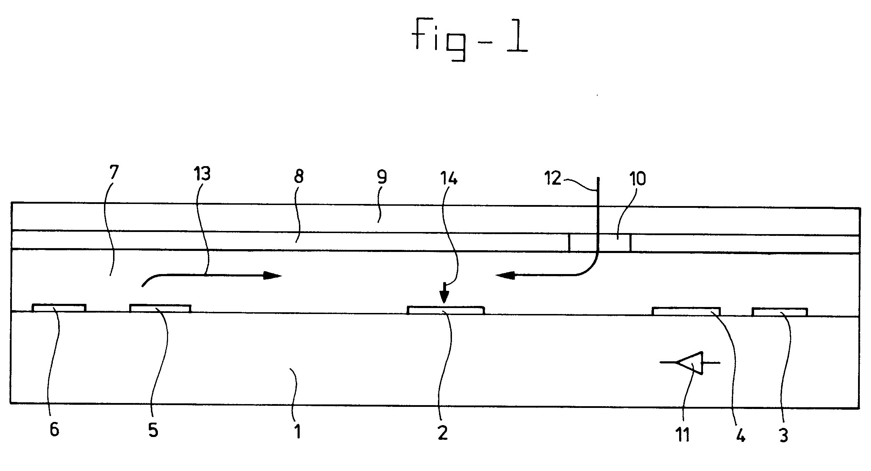

Figure 1 illustrates schematically a cross-section through an embodiment of the sensor according to the invention comprising a substrate 1 which carries on its passivated upper surface a number of electrodes, i.e. the detection electrode 2, a counter electrode 3 cooperating with said detection electrode 2, a reference electrode 4, an oxygen producing electrode 5 and a counter electrode 6 cooperating with said oxygen producing electrode 5. As is illustrated in more detail in the above-mentioned EP 0 396 788 the substrate 1 can be made of silicon and may carry on its upper surface passivation layers consisting of Si₃N₄ and SiO₂. The oxygen producing electrode and the detection electrode can be made of Pt and the reference electrode 4, used for controlling the voltage potential on the detection electrode can be made for instance of Ag/AgCl.

The combination of the substrate 1 with the various electrodes thereon is covered by a hydrogel layer 7 containing the glucose oxidase enzyme G.O.D. The hydrogel layer 7 is covered by a first membrane 8 which is impermeable to glucose. Said membrane 8 comprises a hole or opening 10 through which glucose may pass from the fluid to be measured, which is present above the further membrane 9 in figure 1 through said further membrane 9 into the hydrogel layer 7. The membrane 8 is covered by the already mentioned second membrane 9, which is permeable to oxygen, glucose and water, but impermeable for components with high molecular weight in the glucose containing fluid to be measured. If said fluid to be measured is blood, then the membrane 8 is for instance impermeable to the red blood cells and proteins.

The illustrated embodiment comprises furthermore a temperature measuring element, which is schematically indicated as a diode 11. The functioning of such a temperature sensing element in this type of sensors is considered to be known to the expert in this field. Further information about its function can be found in the above-mentioned references.

As is indicated by the arrow 12, during operation glucose from the fluid, such as blood, which is present above the membrane 9 will diffuse through the membrane 9, will thereafter pass through the hole 10 in the membrane 8, and will reach the hydrogel layer 7. In the hydrogel layer 7 the glucose diffuses in the direction of the detection electrode 2. Simultaneously oxygen is produced by means of the oxygen producing electrode 5 in combination with its counter electrode 6 and the produced oxygen starts diffusing from the electrode 5 in the direction of the detection electrode 2 as is schematically illustrated by the arrow 13. Glucose will diffuse through the hydrogel layer 7 at a lower rate than oxygen. Therefore the distance between the hole 10 and the detection electrode 2 is selected smaller than the distance between the oxygen producing electrode 5 and the detection electrode 2 such, that the glucose flow 12 will meet the oxygen flow 13 right above the detection electrode 2. As a consequence the reaction between the glucose 12 and the oxygen 13 will take place in a defined limited area just above the detection electrode 2 and as a consequence the hydrogen peroxide, formed in said reaction, can be directly oxidized or reduced by the detection electrode 2 resulting into a clearly detectable electrical current through said detector 2.

The detection of hydrogen peroxide can be performed by oxidization or reduction. If hydrogen peroxide is oxidized at the detection electrode then oxygen is created which from the detection electrode will diffuse into the hydrogel layer 7. This oxygen flow will lead to a disturbance of the concentration profiles of glucose, oxygen en hydrogen peroxide and will result into a shift of the reaction zone into the direction of the hole 10 in the membrane 8. That implies, that the rate at which the oxygen is formed at the detection electrode should be maintained as small as possible so that the shift of the reaction zone is minimized. The reduction of hydrogen peroxide at the detection electrode produces water. This has not significant influence on the location of the reaction zone. Because the concentration of oxygen in the reaction zone is small the hydrogen peroxide will almost exclusively be reduced at the detection electrode if the voltage potential thereof is selected at a suitable level.

To be able to determine the eventual contribution of oxygen to the reduction current it is possible to select during a relatively short time period the voltage potential such that the hydrogen peroxide is exclusively oxidized. During this short time period the concentration gradiant of hydrogen peroxide at the detection electrode has to be maintained constant. The oxidation and reduction of the hydrogen peroxide are carried out at a voltage potential whereby the concentration of the hydrogen peroxide at the electrode surface is nearly 0.

During longer use hydrogen peroxide might accumulate in the glucose oxidase containing hydrogel layer 7 resulting into a decrease of the catalytic activity of the G.O.D. This accumulation can be prevented by oxidizing or reducing all the hydrogen peroxide which is formed within the sensor or by transporting the remaining hydrogen peroxide out of the sensor. This last option can be obtained by using only membranes which are permeable to hydrogen peroxide. Because these membranes are in most cases also permeable to oxygen at least part of the oxygen formed in the sensor will disappear out of the sensor without any reaction with the glucose. The operation principle of the hereby described new sensor is not influenced thereby.

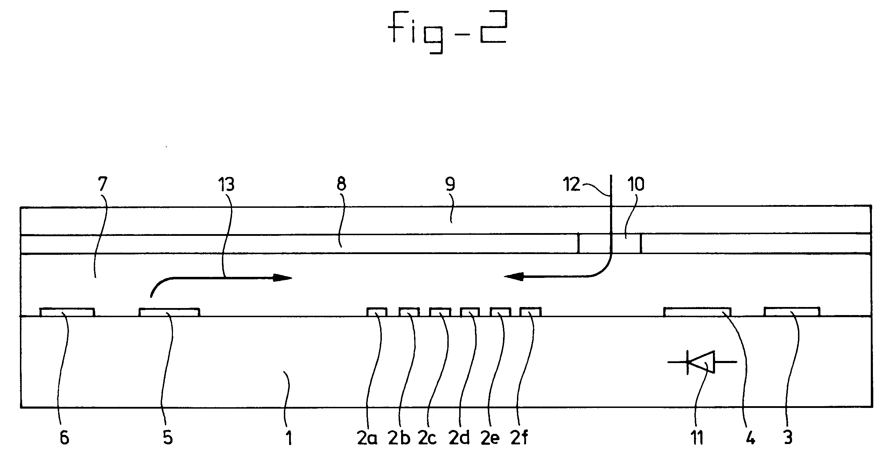

Figure 2 illustrates a further developed embodiment of the glucose sensor according to the invention. The same reference numbers are used as in figure 1 for corresponding components. The only difference between the embodiments illustrated in figures 1 and 2 resides in the configuration of the detection electrode 2. In figure 1 only one detection electrode 2 was used whereas in figure 2 the detection electrode is subdivided into a number of subelectrodes 2a, 2b, 2c, 2d, 2e, 2f. The reaction zone in which glucose reacts with oxygen is not restricted to exactly one very limited zone, but has a certain width. The width of the reaction zone in which the hydrogen peroxide is formed, is dependent onto the catalytic activity of the G.O.D. enzyme and is dependent on the diffusion rates of oxygen and glucose. It will be readily understood that stationary concentration profiles for oxygen, glucose and hydrogen peroxide will develop during operation of the sensor. To obtain more information about this concentration profiles the detection electrode can be subdivided into a number of subelectrodes as illustrated in figure 2. These subelectrodes are formed as an array of electrodes covering the whole width of the reaction zone. The various electrical currents running through these subelectrodes during operation of the sensor provide information about the concentration profile of the hydrogen peroxide in the whole reaction zone.

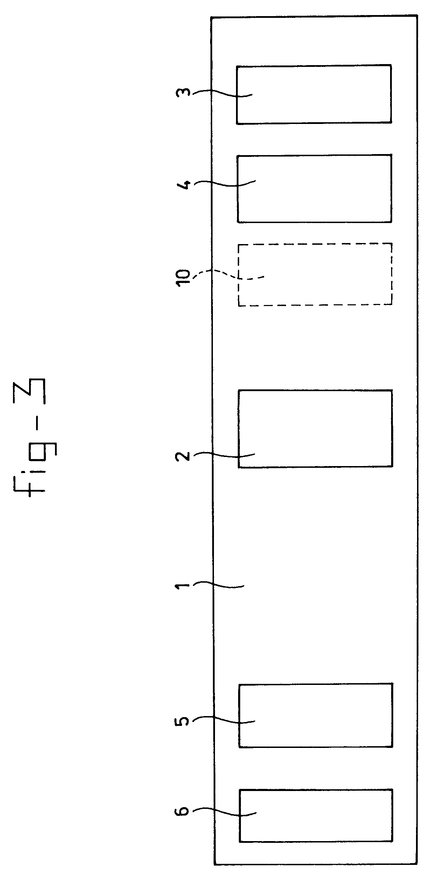

Finally figure 3 illustrates a schematic top view on a part of the sensor whereby the membranes 8 and 9 and the hydrogel layer 7 are removed to show the configuration of the various electrodes 2, 3, 4, 5 and 6 on the passivated top surface of the substrate 1. With dashed lines the location of the hole 10 in relation to the position of the various electrodes is shown in figure 3.

## Claims

1. Electrochemical sensor for measuring the glucose content fluids, containing very low oxygen content or no oxygen at all, comprising:
- a substrate (1) carrying on its passivated surface
- a detection electrode (2)
- a counter electrode (3) cooperating with said detection electrode (2)
- a reference electrode (4)
- a glucose oxidase (G.O.D) containing hydrogel layer (7) covering said surface of the substrate (1) respectively the electrodes arranged thereon
- a membrane (8) impermeable to glucose, having an opening or hole (10) rendering said membrane locally permeable for glucose
- a further membrane (9) permeable to oxygen, glucose and water but impermeable for components with high molecular weight in the glucose containing fluid
characterized in that, the substrate (1) carries furthermore
- an oxygen producing electrode (5)
- a second counter electrode (6) cooperating with said oxygen producing electrode (5),
whereby the arrangement of the various electrodes on the substrate (1) surface and the location of the hole (10) in said membrane (8) is such that the direction of the glucose diffusion path (12) between said hole (10) and the detection electrode (2) is opposite to the direction of the oxygen diffusion path (13) between the oxygen producing electrode (5) and the detection electrode (2).

2. Electrochemical sensor according to claim 1, characterized in that the detection electrode is embodied as an array of subelectrodes (2a, ...., 2f) extending with predetermined mutual distance perpendicular to the glucose and oxygen diffusion directions (12, 13).
